# EUROPEAN PATENT SPECIFICATION

(11) **EP 1 627 680 B1**
(45) Date of publication and mention of the grant of the patent: **21.05.2008**
(21) Application number: 05017784.9
(22) Date of filing: 16.08.2005
(51) Int. Cl.: B01J 19/00

(54) **Inkjet spotting apparatus and method for manufacturing microarrays**
Tintenstrahlbetupfungsgerät und Verfahren zur Herstellung von Micromatrizen
Dispositif du type jet d'encre et procédé de dépôt de microréseaux

(30) Priority: 17.08.2004 KR 2004064590
(43) Date of publication of application: 22.02.2006
(73) Proprietor: SAMSUNG ELECTRONICS CO., LTD., Suwon-si Gyeonggi-do 442-742 (KR)
(72) Inventor: Lim, Ji-hyuk, Yeongtong-gu Suwon-si Gyeonggi-do (KR); Sohn, Dong-kee, Yeongdeungpo-gu Seoul (KR); Kim, Min-soo, Seocho-gu Seoul (KR); Kuk, Keon, Yongin-si Gyeonggi-do (KR)
(74) Representative: Grünecker, Kinkeldey, Stockmair & Schwanhäusser Anwaltssozietät

(56) References cited:
- EP-A- 1 225 472
- WO-A-97/15394
- US-A1- 2003 111 599
- US-A1- 2004 021 068
- US-A1- 2004 206 902

## Description

### BACKGROUND OF THE INVENTION

### 1. Field of the Invention

The present invention relates to an inkjet spotting apparatus for manufacturing microarrays and a method of spotting using the same.

### 2. Description of the Related Art

Microrrays or biochips are microchips which have biomolecules, such as probe DNAs or proteins, immobilized at a high density on predetermined regions of a solid substrate thereof. Microarrays play a very important role in bioengineering fields including diagnosis of diseases, development of new drugs, identification of nucleic acid sequences, etc.

A conventional method of producing microarrays comprises contacting a surface of a solid support with a pin containing a biomolecule solution. However, in such a method, a tip of the pin and the surface of the solid support are deformed due to physical contact, and thus, uniformity of the microarrays is deteriorated. Recently, apparatuses for manufacturing microarrays which spot a biomolecule solution on a solid support in a non-contact manner using inkjetting have been developed.

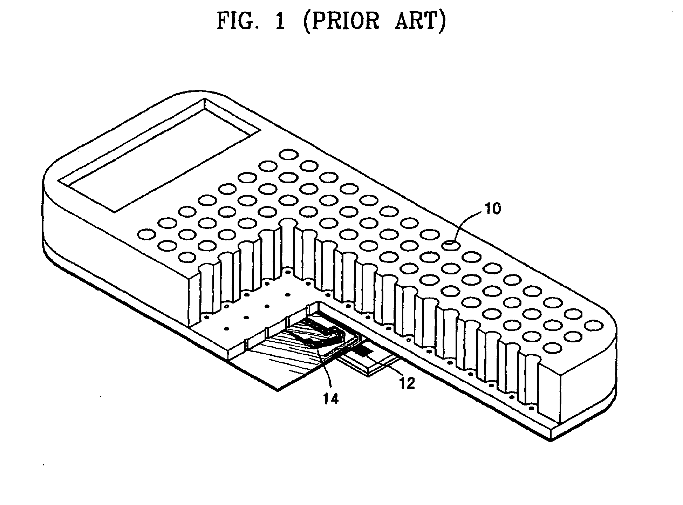

FIG. 1 is a partial cut-away perspective view of a conventional inkjet spotting apparatus for manufacturing microarrays. Referring to FIG. 1, the conventional spotting apparatus comprises a plurality of reservoirs 10 which are filled with a predetermined biomolecule solution which is injected from the outside, a plurality of nozzles 12 through which the biomolecule solution is ejected, and a plurality of microchannels 14 connecting the reservoirs 10 to the nozzles 12. The distancees between the nozzles 12 are equivalent to the distancees between spots in the microarray.

However, in the conventional spotting apparatus, the size of the reservoirs 10 (several mm) are much larger than size of the nozzles 12 (about 20 µm) and the distancees between the reservoirs 10 (several mm) are much larger than the distancees between the nozzles 12 (about 150 µm). Thus, the microchannels 14 connecting the reservoirs 10 to the nozzles 12 are very complicated and long. As a result, it is difficult for the biomolecule solution to be supplied from the reservoirs 10 to the nozzles 12 with ease.

WO-A-97/15394 discloses a microwell plate with corresponding nozzles and well, always arranged in the same relation, which means with the same distance therebetween.

US-A-2004/0021068 discloses an interface member and holder with a number of channels and nozzles and a particular spacing therebetween.

US 2003/0111599 discloses another microfluidic array device, according to which corresponding microfluidic channels or nozzles are also arranged in the same distance without any variations in one of the directions of arrangements.

### Summary of the Invention

It is an object of the invention to provide a spotting apparatus for manufacturing micro arrays having a simplified channel structure that simultaneously allows an easy supply of, for example, biomolecule solution from a reservoir to the nozzles. Moreover, the manufacturing process is simplified, and the yield is increased.

This object is solved by the features of the independent claims.

Advantageous embodiments are disclosed by the sub claims.

According to the method of the invention, the biomolecule solution is ejected sequentially from the nozzles in a row on the solid support, the spotting apparatus moves in the first direction to form a spot column in the second direction on the solid substrate. The second direction may be perpendicular to the first direction.

### Brief Description of the Drawings

The above and other features and advantages of the present invention will become more apparent by describing in detail exemplary embodiments thereof with reference to the attached drawings in which:
FIG. 1 is a partial cut-away perspective view of a conventional spotting apparatus for manufacturing microarrays;
FIG. 2 is a partial top view of a spotting apparatus for manufacturing microarrays according to an embodiment of the present invention;
FIG. 3 is a cross-sectional view taken along line III-III' of the apparatus illustrated in FIG. 2;
FIG. 4 is a top view of the spotting apparatus for manufacturing microarrays illustrated in FIG. 2;
FIG. 5 is a view illustrating spot columns formed by a biomolecule solution ejected from the spotting apparatus illustrated in FIG. 4; and
FIGS. 6A through 6H are views illustrating a method of manufacturing microarrays using the spotting apparatus according to an embodiment of the present invention.

### Detailed Description Of The Invention

Hereinafter, exemplary embodiments of the present invention will be described in detail with reference to the attached drawings. Throughout the drawings, like reference numerals denote like elements. The first direction and the second direction described above will be exemplified as an x-direction and a y-direction in the drawings, respectively.

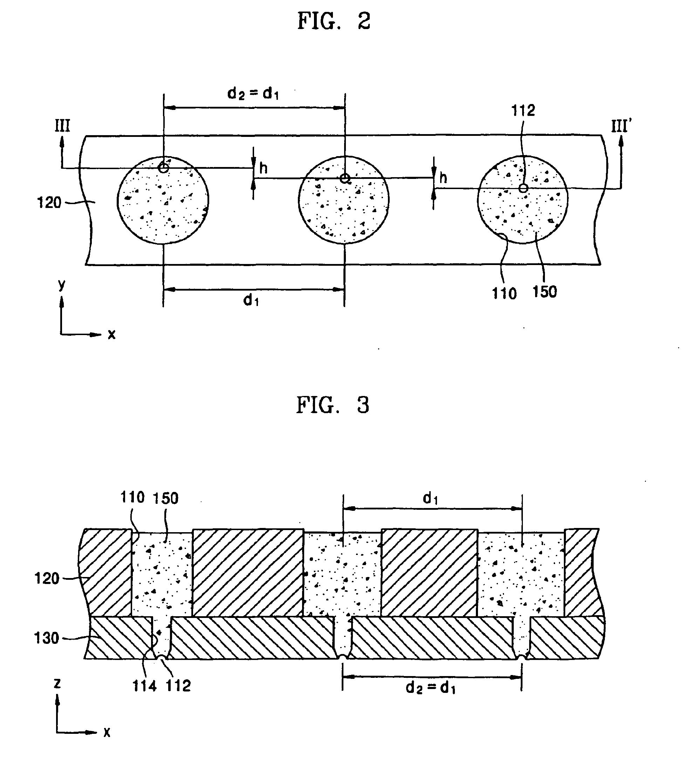

FIG. 2 is a partial top view of a spotting apparatus for manufacturing microarrays according to an embodiment of the present invention. FIG. 3 is a cross-sectional view taken along line III-III' of the apparatus illustrated in FIG. 2.

Referring to FIGS. 2 and 3, the spotting apparatus for manufacturing microarrays according to an embodiment of the present invention includes a first substrate 120 and a second substrate 130 which the first substrate 120 is formed on. The first substrate 120 may be a glass substrate and the second substrate 130 may be a silicon wafer. The first substrate 120 and the second substrate 130 may be manufactured in an integrated structure.

A plurality of reservoirs 110 are formed in the first substrate 120. The reservoirs 110 are filled with a predetermined biomolecule solution 150, which is injected from the outside. Each of the reservoirs 110 may have a circular cross-section as illustrated in FIG. 2 or a quadrangular or hexagonal cross-section, etc. Each of the reservoirs 110 may have a diameter of several mm. The reservoirs 110 are arranged separated by a predetermined distance d₁ along the x-direction. The distance d₁ between the reservoirs 110 may be several mm, preferably 1-5 mm.

A plurality of nozzles 112 through which the biomolecule solution 150 is ejected are formed in a bottom portion of the second substrate 130. The nozzles 112 correspond to the reservoirs 110. Each of the nozzles 112 may have a diameter of about 20 µm. A plurality of channels 114 connecting the reservoirs 110 to the nozzles 112 are formed in a top portion of the second substrate 130.

The nozzles 112 are arranged inclined to the x-direction at a predetermined angle. A distance d₂ between the nozzles 112 in the x-direction is larger than a distance between spots in a spot array to be formed on the microarray. The distance d₂ between the nozzles 112 in the x-direction may be substantially the same as the distance d₁ between the reservoirs 110, as illustrated in FIGS. 2 and 3. Thus, in this case, the distance d₂ between the nozzles 112 in the x-direction may be several mm, preferably 1-5 mm. When the distance d₂ between the nozzles 112 in the x-direction is the same as the distance d₁ between the reservoirs 110, the channels 114 may be shortened and have the more simplified structure, and thus, the biomolecule solution 150 may be supplied from the reservoirs 110 to the nozzles 112 through the channels 114 with ease. A distance h between the nozzles 112 in the y-direction may be substantially the same as the distance between the spots. In this case, the distance h between the nozzles 112 in the y-direction may be 30-300 µm.

The spotting apparatus for manufacturing microarrays according to an embodiment of the present invention ejects the biomolecule solution using an inkjet method. The inkjet method may be a thermal, piezoelectric, or electrostatic inkjet method.

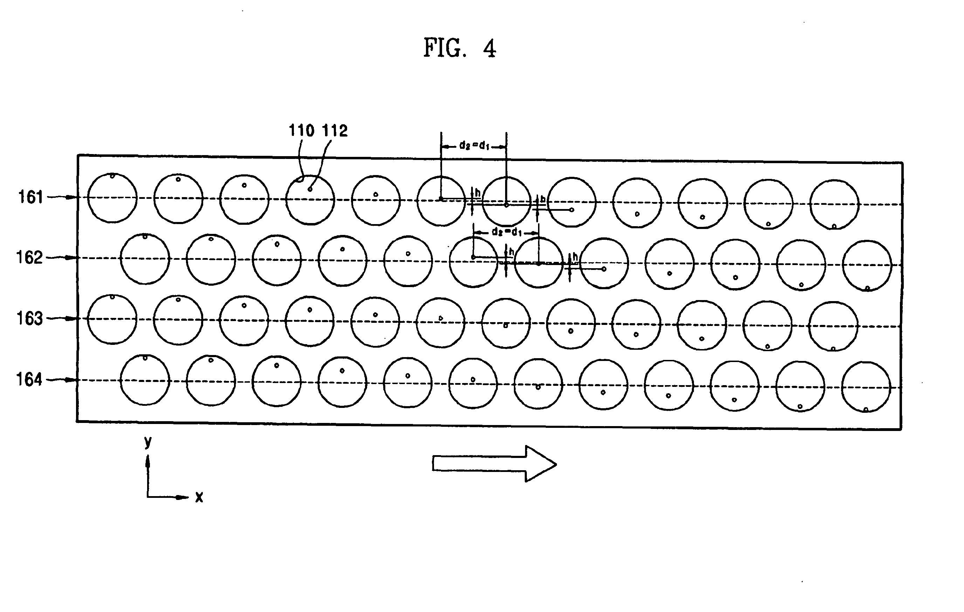

FIG. 4 illustrates the inkjet-type spotting apparatus for manufacturing microarrays, which adopts the arrangement of reservoirs and nozzles as illustrated in FIGS. 2 and 3. Referring to FIG. 4, the reservoirs 110 are arranged in four rows, i.e., a first reservoir row 161, a second reservoir row 162, a third reservoir row 163, and a fourth reservoir row 164. Each of the first through fourth reservoir rows 161, 162, 163, and 164 includes twelve of the reservoirs 110 arranged separated by the distance d₁ in the x-direction. The nozzles 112 are arranged in four rows, the nozzles 112 corresponding to the reservoirs 110. In this case, twelve of the nozzles 112 which constitute a row are arranged to be inclined to the x-direction by a predetermined angle. The distance d₂ between the nozzles 112 in the x-direction may be the same as distance d₁ between the reservoirs 110. The distance h between the nozzles 112 in the y-direction may be the same as the distance between the spots in the microarray to be manufactured.

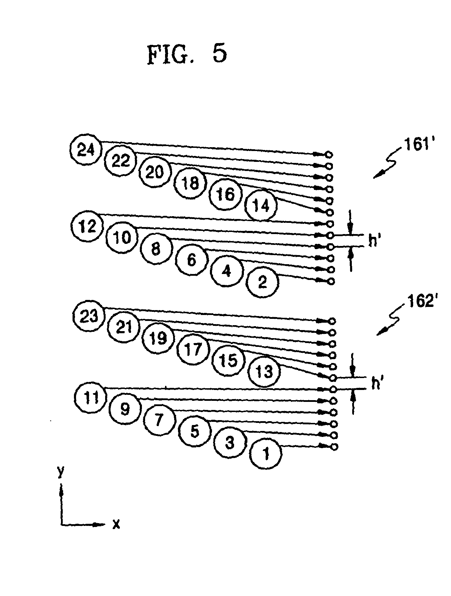

The spotting apparatus having this structure produces a spot array by ejecting the biomolecule solution sequentially from the nozzles 112 on the solid support. Specifically, referring to FIG. 4, when the biomolecule solution is ejected on predetermined locations of a solid support sequentially from the nozzles 112 in all of the reservoir rows 161, 162, 163, and 164 at predetermined time intervals while the spotting apparatus moves in the x-direction, spot columns are formed in the y-direction on the spot array. Referring to FIG. 5, the biomolecule solution is ejected sequentially from twenty-four nozzles 112 of the first reservoir row 161 and the second reservoir row 162, thus forming a first spot column 161' and a second spot column 162' in the y-direction. In FIG. 5, reference numerals 1 through 24 indicate the order in which spots are formed. The first spot column 161' and the second spot column 162' correspond to the first reservoir row 161 and the second reservoir row 162, respectively. A distance h' between the spots arranged in each of the first spot column 161' and the second spot column 162' is the same as the distance h between the nozzles 112 in each of the nozzle rows 161 and 162, in the y-direction.

Although FIG. 4 illustrates a spotting apparatus for manufacturing microarrays comprising four reservoir rows with twelve reservoirs being arranged in each reservoir row in the x-direction, the spotting apparatus for manufacturing microarrays according to the present invention is not limited thereto and various changes can be made therein.

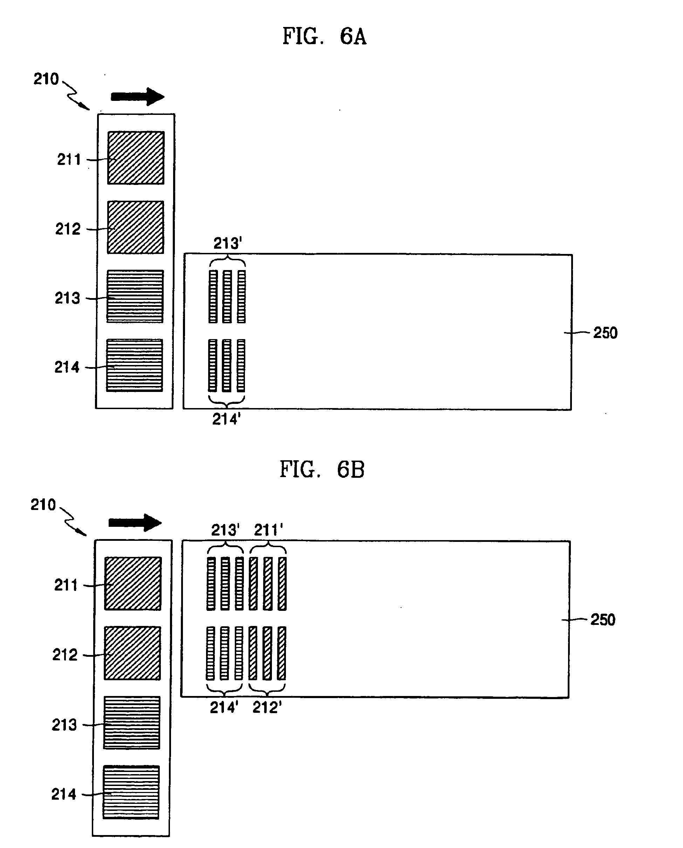

FIGS. 6A through 6H are views illustrating a method of manufacturing microarrays using the spotting apparatus according to an embodiment of the present invention. In FIGS. 6A through 6H, four spotting apparatuses are used to manufacturing a microarray. Each spotting apparatus comprises four units and each unit comprises a row of reservoirs and nozzles corresponding to the respective reservoirs, as described above.

Referring to FIG. 6A, when a biomolecule solution is spotted on a solid support 250 sequentially from a third unit 213 and a fourth unit 214 while a first spotting apparatus 210 moves in an arrow direction, spot columns 213' and 214' which correspond to the third unit 213 and the fourth unit 214, respectively, are formed on predetermined locations of the solid support 250. Next, as illustrated in FIG. 6B, the first spotting apparatus 210 moves down by a predetermined distance such that the first and second units 211 and 212 are aligned with the solid support 250. Then, when the biomolecule solution is spotted on the solid support 250 sequentially from the first unit 211 and the second unit 212 while the first spotting apparatus 210 moves in the arrow direction, spot columns 211' and 212' which correspond to the first unit 211 and the second unit 212, respectively, are formed on predetermined locations of the solid support 250.

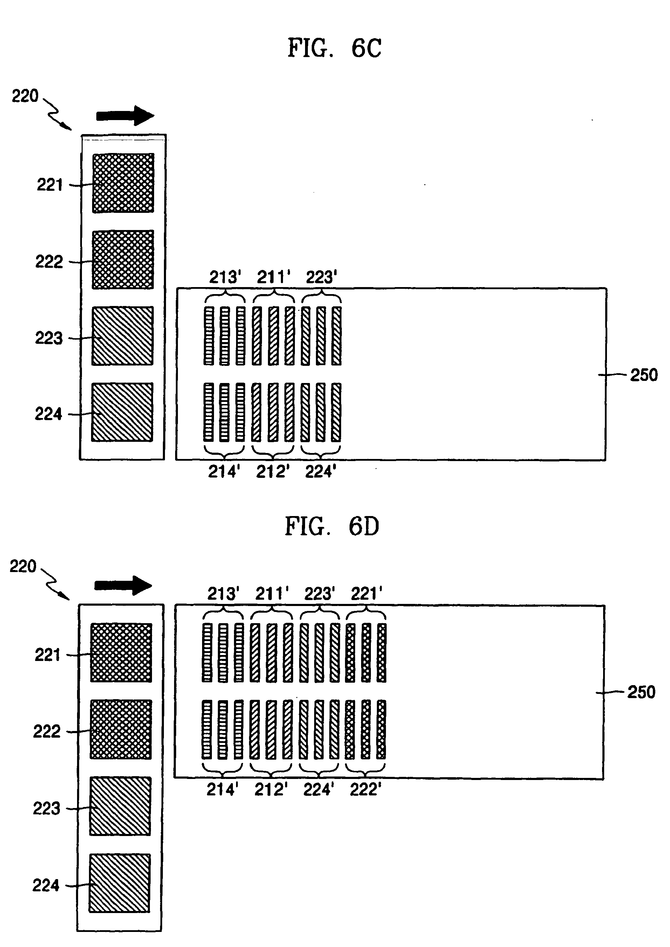

Subsequently, the first spotting apparatus 210 is replaced with a second spotting apparatus 220, and referring to FIG. 6C, while the second spotting apparatus 220 moves in the arrow direction, spot columns 223' and 224' which correspond to a third unit 223 and a fourth unit 224, respectively, are formed on predetermined locations of the solid support 250. Next, as illustrated in FIG. 6D, the second spotting apparatus 220 moves down by a predetermined distance such that the first and second units 221 and 222 are aligned with the solid support 250. Then, while the second spotting apparatus 220 moves in the arrow direction, spot columns 221' and 222' which correspond to the first unit 221 and the second unit 222, respectively, are formed on predetermined locations of the solid support 250.

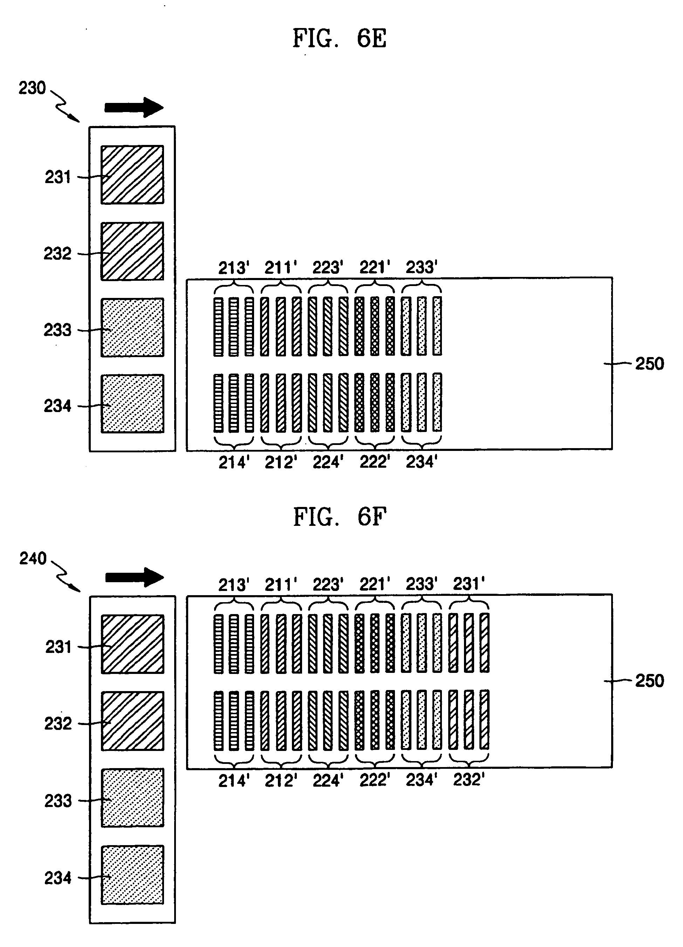

Subsequently, the second spotting apparatus 220 is replaced with a third spotting apparatus 230, and referring to FIG. 6E, while the third spotting apparatus 230 moves in the arrow direction, spot columns 233' and 234' which correspond to a third unit 233 and a fourth unit 234, respectively, are formed on predetermined locations of the solid support 250. Next, as illustrated in FIG. 6F, the third spotting apparatus 230 moves down by a predetermined distance such that the first and second units 231 and 232 are aligned with the solid support 250. Then, while the third spotting apparatus 230 moves in the arrow direction, spot columns 231' and 232' which correspond to the first unit 231 and the second unit 232, respectively, are formed on predetermined locations of the solid support 250.

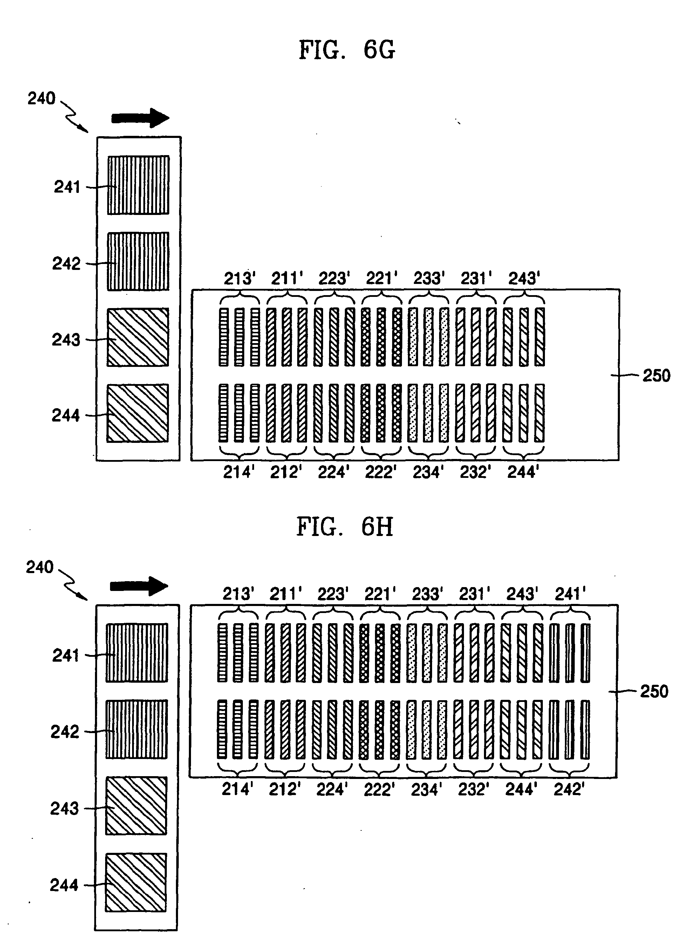

Subsequently, the third spotting apparatus 230 is replaced with a fourth spotting apparatus 240, and as referring to FIG. 6G, while the fourth spotting apparatus 240 moves in the arrow direction, spot columns 243' and 244' which correspond to a third unit 243 and a fourth unit 244, respectively, are formed on predetermined locations of the solid support 250. Next, as illustrated in FIG. 6H, the fourth spotting apparatus 240 moves down by a predetermined distance such that the first and second units 2411 and 242 are aligned with the solid support 250. Then, while the fourth spotting apparatus 240 moves in the arrow direction, spot columns 241' and 242' which correspond to the first unit 241 and the second unit 242, respectively, are formed on predetermined locations of the solid support 250. Thus, a predetermined spot array is formed on the solid support 250 to manufacture the microarray.

The microarray can be manufactured in a relatively short time by using the inkjet spotting apparatus for manufacturing microarrays according to the present invention, as described above. For example, when the spotting apparatus for manufacturing microarrays according to the present invention is used on a 15, 24cm (6-inch) wafer, 96 microarrays each having a size of 12 mm x 12mm can be manufactured within about 10 minutes.

As described above, the spotting apparatus for manufacturing microarrays according to the present invention and the method of spotting using the same have the following effects:

First, the distance between the nozzles is substantially the same as the distance between the reservoirs, and thus, the channels connecting the reservoirs to the nozzles may be shortened and have a more simplified structure. Accordingly, the biomolecule solution may be supplied from the reservoirs to the nozzles with ease.

Second, due to the more simplified structure of the channels, the manufacturing process performed by the apparatus can be simplified and the yield can be increased.

Third, microarrays can be mass-produced by the apparatus in a relatively short time.

## Claims

1. A spotting apparatus for manufacturing microarrays, the spotting apparatus comprising: a plurality of reservoirs (110) which are arranged in rows, and a plurality of nozzles (112) for sequential ejection and adapted to eject while the apparatus is moved in a first direction to form a spot array, **characterized in that** adjacent nozzles (112) are spaced from each other perpendicular to said first directions, such that the distance, d₂, between the nozzles in said first direction is larger than a distance between spots in the spot array.

2. The spotting apparatus of claim 1, wherein the nozzles (112) which constitute a row are arranged to be inclined to the first direction.

3. The spotting apparatus of claim 2, wherein the distance, d₂, between the nozzles (112) in the first direction is substantially the same as a distance between the reservoirs which correspond to the nozzles.

4. The spotting apparatus of claim 3, wherein the reservoirs (110) which correspond to the nozzles are arranged in the first direction.

5. The spotting apparatus of claim 3, wherein the distance, d₂, between the nozzles in the first direction is 1-5 mm.

6. The spotting apparatus of claim 2, wherein a distance between the nozzles of adjacent rows in a second direction is substantially the same as the distance between the spots in the first direction.

7. The spotting apparatus of claim 6, wherein the second direction is perpendicular to the first direction.

8. The spotting apparatus of claim 7, wherein the distance between the nozzles (112) in the second direction is 30-300 µm.

9. The spotting apparatus of claim 1, further comprising a plurality of channels (114) connecting the reservoirs (110) to the nozzles (112).

10. The spotting apparatus of claim 1, comprising: a first substrate (120) having the reservoirs (110); and a second substrate (130) having the nozzles (112).

11. The spotting apparatus of claim 10, wherein the second substrate (130) further has a plurality of channels (114) connecting the reservoirs (110) to the nozzles (112).

12. The spotting apparatus of claim 10, wherein the first substrate (120) is made of glass.

13. The spotting apparatus of claim 10, wherein the second substrate (130) is made of silicon.

14. The spotting apparatus of claim 1, wherein the reservoirs (110) have a circular, quadrangular or hexagonal cross-section.

15. A method of spotting using the spotting apparatus for manufacturing microarrays according to one of the previous claims, said method being **characterized in that** said plurality of reservoirs (110) are filled with a predetermined biomolecule solution; which is ejected through said plurality of nozzles (112) each corresponding to one of the reservoirs (110), wherein the solution is sequentially ejected from the nozzles in each of the rows onto a solid support while the spotting apparatus is moved in said first direction.

16. The method of claim 15, wherein the biomolecule solution is ejected sequentially from the nozzles (112) in a row on the solid support while the spotting apparatus moves in the first direction, to form a spot column in the second direction on the solid substrate.

17. The method of claim 16, wherein a distance between the spots in the spot column is 30-300 µm.

18. The method of claim 15, wherein the biomolecule solution contains nucleic acids or proteins.

19. The method of claim 18, wherein the nucleic acids comprise probe DNAs.

20. The method of claim 15, wherein the biomolecule solution is ejected using an inkjet method.

21. The method of claim 20, wherein the inkjet method is a thermal, piezoelectric, or electrostatic inkjet method.

22. The method of claim 15, comprising spotting by sequentially using a plurality of the spotting apparatuses.

## Patentansprüche

1. Spotting-Vorrichtung zum Herstellen von Microarrays, wobei die Spotting-Vorrichtung umfasst:
eine Vielzahl von Vorratsbehältern (110), die in Reihen angeordnet sind, und eine Vielzahl von Düsen (112) zum sequenziellen Ausstoßen, die so eingerichtet sind, dass sie ausstoßen, während die Vorrichtung in einer ersten Richtung bewegt wird, um ein Spot-Array auszubilden, **dadurch gekennzeichnet, dass** benachbarte Düsen (112) voneinander senkrecht zu der ersten Richtung so beabstandet sind, dass der Abstand d₂ zwischen den Düsen in der ersten Richtung größer ist als ein Abstand zwischen Spots in dem Spot-Array.

2. Spotting-Vorrichtung nach Anspruch 1, wobei die Düsen (112), die eine Reihe bilden, so angeordnet sind, dass sie zu der ersten Richtung geneigt sind.

3. Spotting-Vorrichtung nach Anspruch 2, wobei der Abstand d₂ zwischen den Düsen (112) in der ersten Richtung im Wesentlichen der gleiche ist wie ein Abstand zwischen Vorratsbehältern, die den Düsen entsprechen.

4. Spotting-Vorrichtung nach Anspruch 3, wobei die Vorratsbehälter (110), die den Düsen entsprechen, in der ersten Richtung angeordnet sind.

5. Spotting-Vorrichtung nach Anspruch 3, wobei der Abstand d₂ zwischen den Düsen in der ersten Richtung 1-5 mm beträgt.

6. Spotting-Vorrichtung nach Anspruch 2, wobei ein Abstand zwischen den Düsen benachbarter Reihen in einer zweiten Richtung im Wesentlichen der gleiche ist wie der Abstand zwischen den Spots in der ersten Richtung.

7. Spotting-Vorrichtung nach Anspruch 6, wobei die zweite Richtung senkrecht zu der ersten Richtung ist.

8. Spotting-Vorrichtung nach Anspruch 7, wobei der Abstand zwischen den Düsen (112) in der zweiten Richtung 30-300 µm beträgt.

9. Spotting-Vorrichtung nach Anspruch 1, die des Weiteren eine Vielzahl von Kanälen (114) umfasst, die die Vorratsbehälter (110) mit den Düsen (112) verbinden.

10. Spotting-Vorrichtung nach Anspruch 1, die umfasst:
ein erstes Substrat (120), das die Vorratsbehälter (110) aufweist; und
ein zweites Substrat (130), das die Düsen (112) aufweist.

11. Spotting-Vorrichtung nach Anspruch 10, wobei das zweite Substrat (130) des Weiteren eine Vielzahl von Kanälen (114) aufweist, die die Vorratsbehälter (110) mit den Düsen (112) verbinden.

12. Spotting-Vorrichtung nach Anspruch 10, wobei das erste Substrat (120) aus Glas besteht.

13. Spotting-Vorrichtung nach Anspruch 10, wobei das zweite Substrat (130) aus Silizium besteht.

14. Spotting-Vorrichtung nach Anspruch 1, wobei die Vorratsbehälter (110) einen kreisförmigen, viereckigen oder sechseckigen Querschnitt haben.

15. Verfahren zum Durchführen von Spotting unter Verwendung der Spotting-Vorrichtung zum Herstellen von Microarrays nach einem der vorangehenden Ansprüche, wobei das Verfahren **dadurch gekennzeichnet ist, dass** die Vielzahl von Vorratsbehältern (110) mit einer vorgegebenen Biomolekül-Lösung gefüllt sind, die über die Vielzahl von Düsen (112) ausgestoßen wird, die jeweils einem der Vorratsbehälter (110) entsprechen, wobei die Lösung sequenziell über die Düsen in jeder der Reihen auf einen festen Träger ausgestoßen wird, während die Spotting-Vorrichtung in der ersten Richtung bewegt wird.

16. Verfahren nach Anspruch 15, wobei die Biomolekül-Lösung sequenziell über die Düsen (112) in einer Reihe auf den festen Träger ausgestoßen wird, während sich die Spotting-Vorrichtung in der ersten Richtung bewegt, um eine Spot-Spalte in der zweiten Richtung auf dem festen Substrat auszubilden.

17. Verfahren nach Anspruch 16, wobei ein Abstand zwischen den Spots in der Spot-Spalte 30-300 µm beträgt.

18. Verfahren nach Anspruch 15, wobei die Biomolekül-Lösung Nukleinsäuren oder Proteine enthält.

19. Verfahren nach Anspruch 18, wobei die Nukleinsäuren DNA-Proben umfassen.

20. Verfahren nach Anspruch 15, wobei die Biomolekül-Lösung unter Verwendung eines Tintenstrahlverfahrens ausgestoßen wird.

21. Verfahren nach Anspruch 20, wobei das Tintenstrahlverfahren ein thermisches, piezoelektrisches oder elektrostatisches Tintenstrahlverfahren ist.

22. Verfahren nach Anspruch 15, das Spotting durch sequenziellen Einsatz einer Vielzahl der Spotting-Vorrichtungen umfasst.

## Revendications

1. Dispositif de formation de taches pour fabriquer des microréseaux, le dispositif de formation de taches comprenant : une pluralité de réservoirs (110) qui sont agencés en rangées et une pluralité de buses (112) pour une éjection séquentielle, adaptées pour éjecter tandis que le dispositif est déplacé dans une première direction pour former un réseau de taches, **caractérisé en ce que** les buses adjacentes (112) sont espacées les unes des autres perpendiculairement à ladite première direction, de sorte que la distance, d₂ entre les buses dans ladite première direction est plus grande qu'une distance entre les taches dans le réseau de taches.

2. Dispositif de formation de taches selon la revendication 1, dans lequel les buses (112) qui constituent une rangée sont agencées pour être inclinées dans la première direction.

3. Dispositif de formation de taches selon la revendication 2, dans lequel la distance, d₂, entre les buses (112) dans la première direction est sensiblement la même qu'une distance entre les réservoirs qui correspondent aux buses.

4. Dispositif de formation de taches selon la revendication 3, dans lequel les réservoirs (110) qui correspondent aux buses sont agencés dans la première direction.

5. Dispositif de formation de taches selon la revendication 3, dans lequel la distance d₂, entre les buses dans la première direction est de 1 à 5 mm.

6. Dispositif de formation de taches selon la revendication 2, dans lequel une distance entre les buses de rangées adjacentes dans une seconde direction est sensiblement la même que la distance entre les taches dans la première direction.

7. Dispositif de formation de taches selon la revendication 6, dans lequel la seconde direction est perpendiculaire à la première direction.

8. Dispositif de formation de taches selon la revendication 7, dans lequel la distance entre les buses (112) dans la seconde direction est de 30 à 300 µm.

9. Dispositif de formation de taches selon la revendication 1, comprenant en outre une pluralité de canaux (114) reliant les réservoirs (110) aux buses (112).

10. Dispositif de formation de taches selon la revendication 1, comprenant : un premier substrat (120) comportant les réservoirs (110) ; et un second substrat (130) comportant les buses (112).

11. Dispositif de formation de taches selon la revendication 10, dans lequel le second substrat (130) comporte en outre une pluralité de canaux (114) reliant les réservoirs (110) aux buses (112).

12. Dispositif de formation de taches selon la revendication 10, dans lequel le premier substrat (120) est constitué de verre.

13. Dispositif de formation de taches selon la revendication 10, dans lequel le second substrat (130) est constitué de silicium.

14. Dispositif de formation de taches selon la revendication 1, dans lequel les réservoirs (110) ont une section transversale circulaire, quadrangulaire ou hexagonale.

15. Procédé de formation de taches utilisant le dispositif de formation de taches pour fabriquer des microréseaux selon l'une quelconque des revendications précédentes, ledit procédé étant **caractérisé en ce que** ladite pluralité de réservoirs (110) sont remplis d'une solution de biomolécule prédéterminée qui est éjectée à travers ladite pluralité de buses (112) correspondant chacune à l'un des réservoirs (110), dans lequel la solution est séquentiellement éjectée des buses dans chacune des rangées sur un support solide tandis que le dispositif de formation de taches est déplacé dans ladite première direction.

16. Procédé selon la revendication 15, dans lequel la solution de biomolécule est éjectée séquentiellement des buses (112) dans une rangée sur le support solide tandis que le dispositif de formation de taches se déplace dans la première direction, pour former une colonne de taches dans la seconde direction sur le substrat solide.

17. Procédé selon la revendication 16, dans lequel une distance entre les taches dans la colonne de taches est de 30 à 300 µm.

18. Procédé selon la revendication 15, dans lequel la solution de biomolécule contient des acides nucléiques et des protéines.

19. Procédé selon la revendication 18, dans lequel les acides nucléiques comprennent des ADN sondes.

20. Procédé selon la revendication 15, dans lequel la solution de biomolécule est éjectée en utilisant un procédé à jet d'encre.

21. Procédé selon la revendication 20, dans lequel le procédé à jet d'encre est un procédé à jet d'encre thermique, piézoélectrique ou électrostatique.

22. Procédé selon la revendication 15, comprenant la formation de taches en utilisant séquentiellement une pluralité des dispositifs de formation de taches.
